(19)

Europäisches
Patentamt
European
Patent Office
Office européen
des brevets

(11)    **EP 2 058 358 A1**

(12)    **EUROPEAN PATENT APPLICATION**

(43) Date of publication:
**13.05.2009 Bulletin 2009/20**

(51) Int Cl.:
*C08J 5/22* *(2006.01)*          *G01N 33/50* *(2006.01)*

(21) Application number: **07120468.9**

(22) Date of filing: **12.11.2007**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(71) Applicant: **Koninklijke Philips Electronics N.V.
5621 BA Eindhoven (NL)**

(72) Inventor: **The designation of the inventor has not
yet been filed**

(74) Representative: **Van Velzen, Maaike Mathilde
Philips
Intellectual Property & Standards
P.O. Box 220
5600 AE Eindhoven (NL)**

(54)    **Membranes**

(57)    Subject of the present invention is a porous essentially isotropic polymeric membrane for use in a flow-through/flow-over immunoassay with a high specific surface area, devices comprising such membranes, methods of making them and the use of said membranes and devices in the field of molecular diagnostics.

EP 2 058 358 A1

**Description**

FIELD OF THE INVENTION

The present invention is in the field of molecular diagnostics and life science tool areas. It is in the field of protein determination in an analyte solution, especially quantitative determination of proteins in bodily fluids. The areas of application of the subjects according to the present invention are especially in the field of micro-array biosensors and near patient tests (point-of-care-devices) for molecular diagnostics (MDx). The synthesized membranes can be used in any type of immunoassay:

rapid and sensitive detection of proteins in complex biological mixtures (e.g. blood, saliva, urine, food), for on-site testing and for diagnostics in centralized clinical laboratories.
applications are in medical (sepsis panel, infectious disease and oncology), food and environmental diagnostics and in metabolomics.

BACKGROUND OF THE INVENTION

Lateral flow tests and drug abuse tests are currently being used as point-of-care-diagnostics and have become cheaper and more powerful. Along these same lines, micro-scale protein immunoassays represent a common ground between high-throughput research tools, such as micro-arrays and the diagnostic industry's desire to produce tests which are more efficient. Although protein and antibody array technologies are in the early stage of development, they promise to meet such requirements of research and diagnostics applications.

A micro-array (e.g. for DNA, RNA and proteins) is a tool for making multiple, simultaneous measurements within a small sample. Protein micro-arrays are being utilized for functional proteomic analysis, providing information not obtainable by gene arrays. The micro-array technology is applicable for studying protein-protein, protein-ligand or protein-enzyme activity and posttranslational modifications of proteins. Currently, there are several companies (Schleicher & Schuell, BioSciences, Zyomyx, Cyphergen, Biacore, Perkin Elmer, etc.) manufacturing custom-made protein micro-arrays, which are being primarily used in a research setting. Most of these companies use (modified) solid supports (e.g. glass, plastic, silicon) to immobilize proteins and the sample has to be incubated with the array for a long time, at least a couple of hours, before a detectable signal can be measured. This is not favorable in a clinical setting or an acute care setting where time is of prime essence. To reduce the detection time "flow-through hybridization technology" may be used. (US 5,450,516; US 5,725,949; US 6,214,232; US 6,225,131; US 5,741,647; US 6,020,187; WO 03/004162; WO 2005/077537A1; US 2005/0106583A1; US 6,893,879). The latter has successfully been applied in determining the amount of target nucleic acid present in a sample. For a detailed description of flow-through hybridization technology, capture agents, assays and pumping we refer to the above-mentioned documents. Methods using flow-through hybridization technology while at the same time measuring protein concentrations in analyte solutions are not provided by the prior art. WO 2005/069004 discloses a method of immobilizing proteins in pores that are made of lipid bilayers. This method is especially suitable for detecting binding of molecules to membrane-bound proteins, which are very difficult to obtain in a purified form (the disclosed membrane is a lipid bilayer).

US 6,225,131 discloses an immunoassay performed on a porous aluminum oxide substrate. Incubation of the fluid on the membrane is described.

Proteome Systems has filed a patent disclosing a method and apparatus of a flow through assay for diagnostics purposes. The method entails pre-incubation of the sample with a detection antibody (immobilized onto colloidal gold or a fluorescent tag) to enrich the sample, which is then incubated and captured on a membrane.

The prior art, e.g. WO 2007/069114, further describes arrays of either antigens and/or antibodies that are spotted onto the porous membrane in a defined and ordered pattern. Each spot has a defined position and contains an antibody directed to a defined antigen. The sample will be pumped through the porous membrane a couple of times, allowing for antigen-antibody binding. Antigen-antibody binding will then be optically detected.

Porous polymeric materials are known in the art of polymer technology. Polymer-based monolithic stationary phases for liquid chromatography (LC) were introduced about 15 years ago [Hjerten, S., Liao, J. L., Zhang, R., J. Chromatogr. 1989, 473, 273-275; Tennikova, T. B., Svec, F., Belenkii, B. G., J. Liquid Chromatogr.1990, 13, 63-70; Svec, F., Frechet, J. M. J., Anal. Chem. 1992, 54, 820-822], closely followed by the development of silica-based monoliths [Gusev, I., Huang, X., Horvath, C., J. Chromatogr. A 1999, 69, 4499-4507; Minakuchi, H., Nakanishi, K., Soga, N., Ishizuka, N., Tanaka, N., Anal. Chem. 1996, 68, 3498-3501]. This format represents an alternative to classical packed column technology for both high performance liquid chromatography (HPLC) and capillary electrophoresis chromatography (CEC). The most often emphasized advantages of monolithic stationary phases compared to columns packed with particles are: (i) the simplicity of their in situ preparation from liquid precursors, (ii) the wide range of chemistries available, particularly, for the organic-polymer monoliths, (iii) the possibility to work without retaining frits, since the monolith can be covalently bonded to the surface of the capillary wall or microfluidic device, and (iv) the high column permeability, allowing the use of long columns operating under still moderate pressures to achieve highly efficient separations.

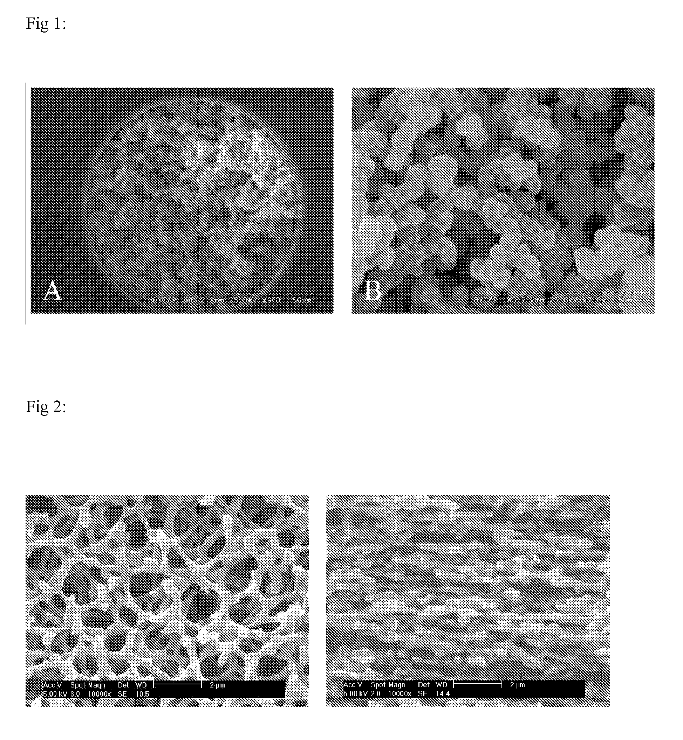

Fig. 1 shows an electron microscope image of a typical polymer-based monolithic stationary phase inside a channel (A) or capillary (B) (S. Eeltink, E.F. Hilder, L. Geiser, F. Svec, J.M.J. Frechet, G.P. Rozing, P.J. Schoenmakers, W.T. Kok, Journal of Separation Science (2007) 30).

Developments in the field of monolithic materials have been well described in numerous reviews [Svec, F., Tennikova, T. B., Deyl, Z. (Eds.), Monolithic Materials: Preparation, Properties, and Applications, Elsevier, Amsterdam 2003; Svec, F., J. Sep. Sci. 2005, 28, 729-745; Svec, F., Huber, C. G., Anal. Chem. 2006, 78, 2100-2107; Allen, D., El Razzi, Z., Electrophoresis 2003, 24, 3962-3976; Rieux, L., Niederlander, H., Verpoorte, E., Bischoff, R., J. Sep. Sci. 2005, 28, 1628-1641]. Most polymer-based monolithic stationary phases are prepared via a single-step copolymerization using a polymerization mixture comprising a monovinyl functional monomer, a divinyl crosslinking monomer, a porogen, and an initiator. The monovinyl monomers provide the monoliths with the required separation chemistry while the porous properties depend on a variety of interrelated factors, such as the type of the monomer, the degree of crosslinking, the composition and concentration of the porogenic solvent, and the temperature [Viklund, C., Svec, F., Frechet, J. M. J., Irgum, K., Chem. Mater. 1996, 8, 744-750; Peters, E. C., Petro, M., Svec, F., Frechet, J. M. J., Anal. Chem. 1998, 70, 2288-2295; Eeltink, S., Herrero-Martinez, J. M., Rozing, G. P., Schoenmakers, P. J., Kok, W. Th., Anal. Chem. 2005, 77, 7342-7347; Courtois, J., Bystom, E., Irgum, K., Polymer 2006, 47, 2603-2611]. All these experimental factors must be optimized coherently together in order to obtain columns with good selectivity, efficiency, and retention.

A typical substrate for protein work, e.g. western blotting and microarrays, is nitrocellulose. Nitrocellulose has a large pore size (~0.45 $\mu$m) and has a network structure. In contrast to the afore-mentioned polymer materials, nitrocellulose is not isotropic, but it is build up in layers.

Fig. 2 shows an electron microscope image of the structure of nitrocellulose.

Nitrocellulose is derived from the biological product cellulose, so the composition of the membranes and especially the pore size cannot be controlled. Therefore, there is an interest for other membranes of which the parameters, such as density, aspect ratio, auto fluorescence, thickness, robustness, and active groups on the inner and outer surfaces can be controlled.

There is a need to provide porous polymeric membranes with a high total specific surface area, preferably with sufficient surface functionalization in a reproducible way, guaranteeing reproducible high recovery and binding efficiencies in micro array immunoassays.

SUMMARY OF THE INVENTION

Subject of the present invention is a porous, essentially isotropic polymeric membrane for use in a flow-through/flow-over immunoassay, where the average total specific surface area is greater than 20 $m^2$/g and wherein the average pore size distribution FWHM (full width at half maximum) is $\leq$ 2 x (twice, two times) the average pore size. In a preferred embodiment the total specific surface area is between 30 and 55 $m^2$/g.

It should be noted that in the context of the present invention, the "total specific surface area" specifically describes the accessible inner and outer surface (e.g. for a fluid) of said porous membrane. Thus, the total specific surface area is the sum of inner and outer surface that is accessible for a fluid.

In another preferred embodiment of the invention the average pore size of the porous essentially isotropic polymeric membrane is between 10 nm and 10 $\mu$m, preferred between 10 nm and 2 $\mu$m, more preferably between 25 nm and 1 $\mu$m, and most preferably between 50 nm and 500 nm.

In a preferred embodiment the membrane thickness is in the range between 10 and 300 $\mu$m, more preferred in the range between 50 and 150 $\mu$m.

The porous essentially isotropic polymeric membrane according to the present invention is obtainable by photo-polymerization induced phase separation of a mixture comprising a cross-linking monomer and a single porogenic solvent, wherein the solvent concentration is between 40 and 80 wt%, preferred between 42 and 70 wt%, more preferred between 45 and 60 wt%, most preferred about 50 wt%.

In another preferred embodiment of the invention the membrane is obtainable by photo-polymerization induced phase separation of said mixture, wherein the cross-linking monomer is tetraethyleneglycoldimethylacrylate. In more preferred embodiments the single porogenic solvent is either polypropyleneglycol or tripropyleneglycol.

In another preferred embodiment of the invention the membrane is obtainable by photo-polymerization induced phase separation of said mixture, wherein the mixture comprises glycidylmethacrylate as further reactive monomer.

It is more preferred that additionally the total specific surface of the resulting polymerized and phase separated membrane furthermore is grafted with a thin layer. "Thin grafted layer" means a layer with a thickness between a few Angström and a few tens of nanometer; it may mean a typical thickness of a monolayer, i.e. in this case a few nanometer. In a preferred embodiment amine groups, and especially so called primary amine groups are functionalized onto the total specific surface. In a preferred embodiment the grafting mixture comprises triamines.

In another preferred embodiment the total specific surface of said porous essentially isotropic polymeric membrane according to the invention is chemically altered resulting in surface functionalization. In a more preferred embodiment the surface chemistry differs locally. In a more preferred embodiment patterning means, such as photo-patterning grafting and/or printing are applied to achieve a surface chemistry which differs locally.

In a further preferred embodiment of the invention capture antibodies are coupled to the surface of said porous essentially isotropic polymeric membrane according to the invention.

Another subject of the invention is a method of making said porous essentially isotropic polymeric membrane comprising the steps of:

- providing a mixture comprising a cross-linking monomer and a single porogenic solvent, wherein the solvent concentration is between 40 and 80 wt%,
- performing a photo-polymerization induced phase separation process applying UV light,
- after completion of polymerization, rinsing the porous membrane with a second solvent, and
- drying of the porous membrane.

In a preferred embodiment of the method of making said membrane the concentration of the single porogenic solvent is between 42 and 70 wt%, more preferred between 45 and 60 wt%, most preferred about 50 wt%.

In another preferred embodiment of the method of making said membrane the cross-linking monomer is tetraethyleneglycoldimethylacrylate. It is further preferred that the single porogenic solvent is either polypropyleneglycol or tripropyleneglycol.

Another preferred embodiment is the method of making said membrane, wherein the mixture comprises glycidylmethacrylate and/or acrylic acid as further reactive monomer.

In a more preferred embodiment of the method of making said membrane the total specific surface of the resulting polymerized and phase separated membrane is furthermore grafted with a thin layer. In an especially preferred embodiment amine groups, and especially primary amine groups are functionalized onto the total specific surface. In one preferred embodiment the grafting mixture comprises triamines such as diethylenetriamine (DETA).

In another preferred embodiment of the method of making said membrane a subsequent post-polymerization step is conducted, wherein the total specific surface of said porous membrane is chemically altered resulting in surface functionalization. In a preferred embodiment the surface chemistry differs locally, which can be achieved by photografting and/or printing.

In an especially preferred embodiment of the method of making said membrane capture antibodies are coupled to the surface of the membrane.

It is a further subject of the invention to provide a device comprising said porous essentially isotropic polymeric membrane.

In another subject of the present invention a micro fluidic bio-separation device is provided comprising a plurality of porous elements made of said porous essentially isotropic polymeric membrane integrated in at least a part of a channel or in multiple parts of multiple channels of said micro fluidic device.

In a preferred embodiment of the device capture antibodies are coupled to the total specific surface of the membrane.

In a preferred embodiment of the device capture antibodies are covalently coupled to the total specific surface of the membrane.

Another embodiment is directed to a device according to the present invention, in which the membrane comprises a plurality of spots, wherein a spot is a region, in which capture antibodies are present / coupled to the membrane. In a preferred embodiment several spots might comprise several capture antibodies, which may be different from one another. Antibodies according to the present invention also encompass fragments thereof as e.g. single chain variable fragments (scFv) and functional sub-parts of antibodies. Capture molecules may also be nucleic acids as e.g. single stranded DNA oligomers.

A further object of the invention is the use of said membrane according to the present invention and the use of a device according to the present invention for molecular diagnostics.

Another object of the invention is the use of said membrane according to the present invention and the use of a device according to the present invention for the detection of proteins in bodily fluids.

A preferred object of the invention is the use of said membrane according to the present invention and the use of a device according to the present invention for performing a quantitative immunoassay for measuring protein concentration in an analyte solution.

Such immunoassay may be e.g. sandwich immunoassays. These immuno assays may be conducted as direct or indirect competitive assays, bridge assays.

BRIEF DESCRIPTION OF THE DRAWINGS

Fig. 1 Scanning Electron microscope image of a typical polymer-based monolithic stationary phase inside a channel (A) or a capillary (B).

Fig. 2 Scanning Electron microscope image of the structure of nitrocellulose: The left image represents the surface; the right image represents the bulk.

Fig. 3 Specific surface area as obtained from BET (Brunauer, Emmet and Teller) adsorption measurements as function of the porogenic solvent concentration (TPG). Values up to about 50 $m^2/g$ have been obtained in the range between 50 and 60% TPG.

Fig. 4 Binding capacity of antibodies onto different membranes. After washing the membranes for two days, the recovery of the membrane with 50% solvent, TEGDMA, GMA, and triamines stayed high.

Fig. 5 Binding efficiency of antibodies onto different membranes.

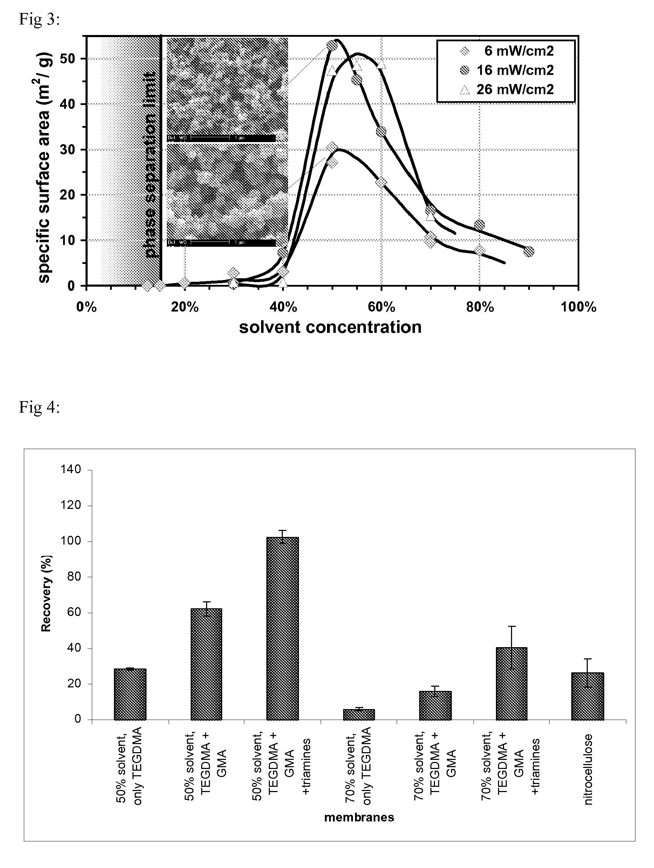

The relative efficiency (in %) i.e. the binding efficiency relative to the nitrocellulose membrane was calculated. It can be clearly derived from the Figure that the efficiency of three of the membranes is about a factor of 3 larger compared to nitrocellose (NC) membrane. Especially the membranes with 50% solvent and with GMA (the same applies to the membrane with additional DETA treatment which are favorable as these membranes are also characterized by large recover as shown in Fig. 4).

DETAILED DESCRIPTION OF EMBODIMENTS

The present invention meets the need to provide porous polymeric membranes with a high specific surface area and preferably sufficient surface functionalization in a reproducible way, guaranteeing reproducible high recovery and binding efficiencies of antibodies and/or of analytes, (allergens, metabolites, protein drugs) in micro array immunoassays.

Thus, subject of the present invention is a porous essentially isotropic polymeric membrane for use in a flow-through/flow-over immunoassay, wherein the average total specific surface area is greater than 20 $m^2/g$ and wherein the average pore size distribution FWHM (full width at half maximum) is $\leq 2$ x (twice, two times) the average pore size. In a preferred embodiment the specific surface area is >30 $m^2/g$, e.g. between 30 and 55 $m^2/g$, more preferably >40 $m^2/g$, most preferred > 50 $m^2/g$, especially preferred about 55 $m^2/g$. This is a very high value for a specific surface area, compared to other porous polymeric materials, which typically have a specific surface area of up to 15 $m^2/g$. Nitrocellose has a specific surface area of about 8 $m^2/g$.

The term "essentially" means and/or includes especially a wt-% content of $\geq 90$ %, according to another embodiment $\geq 95$ %, according to yet another embodiment $\geq 99$ %. Essentially isotropic means that polymeric structures, pores, voids, walls, globulary and/or tubular structures are substantially randomly orientated and distributed. Substantially no alignment and/or lateral ordering are present. Especially fluid flow, and optical properties of said membranes are substantially isotropic, i.e. identical in any special direction.

A membrane according to the present invention is a porous polymer. The term membrane encompasses also the term porous material or porous element.

In another preferred embodiment of the invention the average pore size of the porous essentially isotropic polymeric membrane is between 10 nm and 10 $\mu$m, preferred between 10 nm and 2 $\mu$m, more preferably between 25 nm and 1 $\mu$m, and most preferably between 50 nm and 500 nm. The pore size distribution is preferably very small (FWHM < factor 2 of average pore size).

In a preferred embodiment the membrane thickness is in the range between 10 and 300 $\mu$m, even more preferred in the range between 50 and 150 $\mu$m.

According to a preferred embodiment an at least essential part (>50%) of the outer surface of said porous membrane is covered with open pores.

According to a preferred embodiment said porous membrane has an open porosity of at least $\geq 40$ % and $\leq 80$%, preferred between 42 and 70 %, more preferred between 45 and 60 %, most preferred about 50 %.

(The term "open porosity of X%" specifically means and/or includes that X% of the volume of the porous membrane is empty and it especially includes the pores are connected to each other and to the outer surface of the membrane. In this context, the term "open porosity" especially means or includes the fraction of the total volume in which fluid flow is effectively taking place.)

The porous essentially isotropic polymeric membrane according to the present invention is obtainable by photopolymerization induced phase separation of a mixture comprising a cross-linking monomer and a single porogenic solvent, wherein the solvent concentration is between 40 and 80 wt%, preferred between 42 and 70 wt%, more preferred between 45 and 60 wt%, most preferred about 50 wt%.

Porogenic solvents are those solvents which are suitable for forming pores and/or displacing the polymer chains during polymerization. The characteristics and use of such solvents in the formation of macroreticular or macroporous resins are described in US 4,224,415, which is included herein by reference. A porogenic solvent is one which dissolves the monomer mixture being (co-)polymerized but which does not dissolve the (co-)polymer. In addition, the porogenic solvents must be inert to the polymerization conditions, i.e., neither interfere with or enter into the polymerization.

In a preferred embodiment according to the invention patterned photo-polymerization induced phase separation (PIPS) is used to fabricate the isotropic polymeric membrane.

Preferably the components are chosen such that during said polymerization and phase separation process only negligible UV light scattering occurs in the channel, i.e. almost no turbidity occurs during the patterned polymerization step, which allows to obtain well defined (and well separated) porous elements or membranes.

In another preferred embodiment of the invention the membrane is obtainable by photo-polymerization induced phase separation of said mixture, wherein the cross-linking monomer is tetraethyleneglycoldimethylacrylate. In more preferred embodiments the single porogenic solvent is either polypropyleneglycol or tripropyleneglycol.

In another preferred embodiment of the invention the membrane is obtainable by photo-polymerization induced phase separation of said mixture, wherein the mixture comprises glycidylmethacrylate as further reactive monomer.

It is more preferred that additionally the total specific surface of the resulting polymerized and phase separated membrane furthermore is grafted with a thin layer. In a preferred embodiment amine groups, and especially so called primary amine groups are functionalized onto the total specific surface. In a preferred embodiment the grafting mixture comprises triamines.

Further examples for the cross-linking monomer and the single porogenic solvent which may be used according to the present invention are given below.

According to an embodiment of the present invention, the material mixture of which the porous membrane is made of is a polymeric material comprising a poly(meth)acrylic material.

According to an embodiment of the present invention, the porous membrane comprises a poly(meth)acrylic material made by the polymerization of at least one (meth)acrylic monomer and at least one polyfunctional (meth)acrylic monomer.

According to an embodiment of the present invention, the (meth)acrylic monomer is chosen from the group comprising (meth)acrylamide, (meth)acrylic acid, hydroxyethyl(meth)acrylate, ethoxyethoxyethyl(meth)acrylate, hydroxyethylmeth(meth)acrylate, isobornyl(meth)acrylate, isobornyl meth(meth)acrylate or mixtures thereof.

According to an embodiment of the present invention, the polyfunctional (meth)acrylic monomer is a bis-(meth) acryl and/or a tri-(meth)acryl and/or a tetra-(meth)acryl and/or a penta-(meth)acryl monomer.

According to an embodiment of the present invention, the polyfunctional (meth)acrylic monomer is chosen out of the group comprising bis(meth)acrylamide, tripropyleneglycol di(meth)acrylates, pentaerythritol tri(meth)acrylate, polyethyleneglycoldi(meth)acrylate, ethoxylated bisphenol-A-di(meth)acrylate or mixtures thereof.

According to an embodiment of the present invention, the crosslink density in the poly(meth)acrylic material is $\geq 0.05$ and $\leq 1$.

In the sense of the present invention, the term "crosslink density" means or includes especially the following definition: The crosslink density $\delta_X$ is here defined as $\delta_X = \dfrac{X}{L+X}$ where X is the molar fraction of polyfunctional monomers and L the molar fraction of linear chain (= non polyfunctional) forming monomers. In a linear polymer $\delta_X = 0$, in a fully crosslinked system $\delta_X = 1$.

Glycidylmethacrylate (GMA) can be added to the mixture to provide epoxy groups on the total specific surface of the porous membrane. Preferably the monomer weight ratio of GMA with respect to TEGDMA is in the range between 1:10 and 1:1000, preferably between 1:50 and 1:500. GMA is also a reactive monomer and will be incorporated in the polymer network during photo-polymerization induced phase separation.

Acrylic acid (AA) can be added to the mixture to introduce carboxy groups on the total specific surface of the porous membrane. Preferably the monomer weight ratio of AA with respect to TEGDMA is in the range between 1:10 and 1:1000, preferably between 1:50 and 1:500. AA is also a reactive monomer and will be incorporated in the polymer network during photo-polymerization induced phase separation.

In another preferred embodiment the total specific surface of said porous essentially isotropic polymeric membrane according to the invention is chemically altered resulting in surface functionalization. In a preferred embodiment the surface chemistry differs locally. This means according to another preferred embodiment said polymeric materials have locally different affinity or surface functionality, which is obtained e.g. by local photo-grafting printing patterning as explained in detail e.g. in [S. Eeltink, E.F. Hilder, L. Geiser, F. Svec, J.M.J. Frechet, G.P. Rozing, P.J. Schoenmakers, W.T. Kok, Journal of Separation Science (2007) 30]. A two-step process is a possibility to create polymeric porous membranes with dedicated functionality. It includes the preparation of a "generic" porous membrane with optimized pore structure using a simple homogeneous mixture of monomers and porogenic solvents, followed by adjustment of its surface chemistry in a post-polymerization step. This then leads to polymers with the desired "surface" functionalities attached to the pore surface (see also [Rohr, T., Hilder, E. F., Donovan, J. J., Svec, F., Frechet, J. M. J., Macromolecules 2003, 36, 1677-1684; Rohr, T., Ogletree, F., Svec, F., Frechet, J. M. J., Adv. Funct. Mater. 2003, 13, 264-270; Meyer, U., Svec, F., Frechet, J. M. J., Hawker, C. J., Irgum., K., Macromolecules 2000, 33, 7769-7775]). Using this approach, different surface chemistries have been developed for fabrication of devices for protein mapping with several consecutive chemistries in the same monolith [Peterson, D. S., Rohr, T., Svec, F., Frechet, J. M. J. Anal. Chem. 2003, 75, 5328-5635], and for the preparation of columns with a continuous longitudinal chemical composition gradient [Pucci, V., Raggi, M. A., Svec, F., Frechet, J. M. J., J. Sep. Sci. 2004, 27, 779-788] which are incorporated in the present invention by reference.

In a further preferred embodiment of the invention capture antibodies are coupled to the surface of said porous essentially isotropic polymeric membrane according to the invention. In a preferred embodiment the surface chemistry is tailored locally e.g. by photografting, allowing to perform a quantitative immunoassay without need of printing various antibody concentrations.

The membranes according to the present invention show a well controlled morphology, resulting in very high recovery and binding efficiencies, which is a significant improvement compared to membranes known in the art. Moreover, batch to batch variations were minimal indicating its suitability in the diagnostic field.

Another subject of the invention is a method of making said porous essentially isotropic polymeric membrane comprising the steps of:

- providing a mixture comprising a cross-linking monomer and a single porogenic solvent, wherein the solvent concentration is between 40 and 80 wt%,
- performing a photo-polymerization induced phase separation process applying UV light,
- after completion of polymerization, rinsing the porous membrane with a second solvent, and
- drying of the porous membrane.

In a preferred embodiment of the method of making said membrane the single porogenic solvent concentration is between 42 and 70 wt%, more preferred between 45 and 60 wt%, most preferred about 50 wt%.

In another preferred embodiment of the method of making said membrane the cross-linking monomer is tetraethyleneglycoldimethylacrylate. It is further preferred that the single porogenic solvent is either polypropyleneglycol or tripropyleneglycol. In another preferred embodiment of the method of making said membrane the mixture comprises glycidylmethacrylate and/or acrylic acid as further reactive monomer.

In a more preferred embodiment of the method of making said membrane the total specific surface of the resulting polymerized and phase separated membrane is furthermore grafted with a thin layer. In an especially preferred embodiment amine groups, and especially so called primary amine groups are functionalized onto the total specific surface. In one preferred embodiment the grafting mixture comprises triamines.

In another preferred embodiment of the method of making said membrane a subsequent post-polymerization step is conducted, wherein the total specific surface of said porous membrane is chemically altered resulting in surface functionalization. In a more preferred embodiment the surface chemistry differs locally, which can be achieved by photografting printing, patterning.

The surface of the pores may have surface groups which are reactive groups such as epoxy, alcohol, acetal, aldehyde, chloromethyl, thiol, amine, ester, carboxylic acid and anhydride, amide, oxime, imine, hydrazone, enamine, or oxazoline groups. The reactive groups within the pores determine the reactivity of the surfaces thereof.

The process of the present invention selectively modifies the pores of the porous material by employing a modifying agent which reacts with the reactive groups in certain pores, or catalyzes their reaction with another reagent, to chemically modify the reactive surface groups to different surface groups, thereby changing the surface functionality of the pore surface. For example, hydrophilic reactive groups can be changed to hydrophobic groups and vice-versa, changing the surface functionality of the pore surface. Selective modification is achieved by using a modifying agent such as a catalyst or reagent which is of a size which permits it to penetrate only into pores of certain size. Once it penetrates into the pores in which it fits, the chemical modification occurs, transforming the reactive groups therein to surface groups of a different functionality than the original reactive groups. The pores into which the modifying agent cannot penetrate because of size constraints remain unmodified. The resultant porous material contains pores with different surface functionalities. The material is permeable to air and liquids both before and after modification.

The process of the present invention also includes the preparation of materials possessing different reactive groups in pores of at least two size ranges by a series of consecutive reactions using modifying agents with different molecular sizes. In this way, a porous material may be produced containing two or more different surface functionalities localized in pores of different sizes.

In an especially preferred embodiment of the method of making said membrane capture antibodies are coupled to the surface of the membrane.

In a preferred embodiment according to the invention a suitable monomer mixture is filled between a patterned mold and a substrate, and is subsequently photopolymerized with induced phase separation (PIPS). By removing the mold and rinsing the structures, the porous elements (such as, e.g. pillars) attached to the substrate are fabricated.

The molds (with small features and high aspect ratio) can be obtained by reactive ion etched silicon, etched glass, SU-8 patterns on glass, PDMS molds, or other replicates.

It is a further subject of the invention to provide a device comprising said porous essentially isotropic polymeric membrane.

In another subject of the present invention a micro fluidic bio-separation device is provided comprising a plurality of porous elements made of said porous essentially isotropic polymeric membrane integrated in at least a part of a channel or in multiple parts of multiple channels of said micro fluidic device. Thus, in a preferred embodiment a micro fluidic bio-separation device is provided comprising a plurality of porous elements (such as pillars, walls, bumps, posts, obstacles etc.) integrated in at least a part of a channel or in multiple parts of multiple channels of said micro fluidic device. In contrast to prior art said porous elements are substantially made out of polymeric material (e.g. polyacrylates etc.) and are preferably attached to at least one wall of said micro fluidic channel. The inventive device can be a biosensor.

In a preferred embodiment of the device capture antibodies are coupled to the total specific surface of the membrane.

A further object of the invention is the use of said membrane according to the present invention and the use of a device according to the present invention for molecular diagnostics.

Another object of the invention is the use of said membrane according to the present invention and the use of a device according to the present invention for the detection of proteins in bodily fluids.

A preferred object of the invention is the use of said membrane according to the present invention and the use of a device according to the present invention for performing a quantitative immunoassay for measuring protein concentration in an analyte solution.

The devices according to the present invention can be used with flow-through binding (hybridization/incubation) technology. Alternatively flow-over technology can be used. To reduce the detection time it is preferred to use a "flow-through hybridization technology" (described in and incorporated herein by reference US 5,450,516; US 5,725,949; US 6,214,232; US 6,225,131; US 5,741,647; US 6,020,187; WO 03/004162; WO 05/077537 A1; US 2005/0106583 A1; US 6,893,879 B2).

Subject of the present invention is an improved device comprising the porous membrane/material according to the invention. The device may be used for conducting a multiplicity of individual and simultaneous binding reactions or for performing other separation techniques. Capture probes as e.g. antibodies may be attached to the surface of the porous membrane according to the present invention.

The device in accordance with the invention may be functionalized for DNA or protein attachment via epoxy chemistry methods, dextran with terminal epoxide/aldehyde/carboxy functions, by direct post cure activation of the polymer, or by other methods known in the art. In addition, a metallic coating of a noble metal, for example gold, may be applied to the porous device according to the invention. Sputtering or other available methods may be used to apply this coating. The gold coating may be used to vary the opacity of the porous device or to facilitate other conventional molecular attachment methods which may be employed in accordance with the invention. Antibodies are covalently coupled to said porous membrane either via the NHS chemistry, aldehyde or carbohydrate chemistries. These are standard antibody immobilization techniques.

Attachment chemistry using dextran-agarose supports is described in Penzol et al, Biotechnol. Bioeng. 60 (1998) pp. 518-523, included herein by reference.

Binding reagents may be applied to a particular region of the porous device in one step or may be synthesized in situ as is well known in the art. Ink-jet technology may be used to accurately deposit material in a predetermined pattern as is well known in the art and described in WO 95/11755, US2005/0069937 and US2006/0003381, which are included herein by reference.

Ordinarily, the porous device in accordance with the invention is used in conjunction with a known detection technology particularly adapted to enable for discrimination between regions and/or spots of the membrane in which binding has taken place and those in which no binding has occurred. The extent of binding in said different regions and/or can be quantified. In DNA and RNA sequence detection, autoradiography and optical detection are advantageously used. Alternatively, phosphor imager detection methods may be used.

Optical detection of fluorescent-labeled receptors is also employed in detection.

A highly preferred method of detection is a charge-coupled-device array or CCD array. With the CCD array, an individual pixel or group of pixels of the CCD array is placed adjacent to each confined region of the substrate where detection is to be undertaken. Light attenuation, caused by the higher absorption in test sites with hybridized molecules, is used to determine the sites where hybridization has taken place. Lens-based CCD cameras can also be used.

Alternatively, a detection apparatus can be constructed such that sensing of changes in conductance or the dissipation of a capacitor placed contiguous to each confined region can be measured. Similarly, by forming a transmission line between two electrodes contiguous to each confined region hybridized molecules can be measured by the radio-frequence (RF) loss. The preferred methods for use herein are described in WO 93/22678, incorporated herein by reference.

Hybridization or binding conditions and/or techniques e.g. oligonucleotides may be coupled to membranes via UV-cross linking and depending on the annealing temperature and hybridization conditions various DNA strands may be hybridized. In the case of protein detection, antigen:antibody binding is rather specific and thus unbound and/or unspecifically bound molecules are removed by stringent washing procedure. Examples may vary according to the specific probes and/or target molecules and are well known in the art.

The areas of application of the subjects according to the present invention are in the field of micro-array biosensors for molecular diagnostics (MDx):

rapid and sensitive detection of proteins in complex biological mixtures (e.g. blood, saliva, urine, food), for on-site testing and for diagnostics in centralized clinical laboratories.

applications are in medical (sepsis panel, infectious disease and oncology), food and environmental diagnostics and in metabolomics and life science tool area.

Such imunnoassays may be e.g. sandwich immunoassays. These immuno assays may be conducted as direct or indirect competitive assays, bridge assays.

As an example such methods of detecting antigens in analytes are detailed below.

Thus, a further embodiment of the invention is a method of detecting a protein in a bodily fluid comprising the steps of:

- the analyte potentially comprising the antigene is exposed to the total specific surface area of the porous membrane
- in case of the presence of an antigene said antigene is captured by a capture probe, e.g. capture antibody, that is immobilized to said porous membrane
- a further labeled antibody is bound to the captured antigene
- the signal of the label is detected.

Washing steps may be conducted between the specific above-mentioned method steps.

Another embodiment of the invention is a method of detecting a protein in a bodily fluid comprising the steps of:

- a labeled antibody is added to a analyte potentially comprising the antigene
- in case of the presence of an antigene the labeled antibody binds to this antigene
- said analyte is exposed to the total specific surface area of the porous membrane
- in case of the presence of an antigene said antigene is captured by a capture probe, e.g. capture antibody, that is immobilized to said porous membrane
- the signal of the label is detected.

Washing steps may be conducted between the specific above-mentioned method steps.

The above-described methods may be modified and may be conducted as a competition assay. Thus, a further embodiment of the invention is a method of detecting a protein in a bodily fluid comprising the steps of:

- the analyte potentially comprising the antigene is exposed to the total specific surface area of the membrane
- in case of the presence of an antigene said antigene is captured by a capture probe, e.g. capture antibody, that is immobilized to said porous membrane
- a labeled antibody and an unlabelled antibody are added and compete for the binding to the antigene
- The signal of the label is detected. The signal intensity is inversely proportional to the amount of analyte present in the sample

Thus the present invention provides a rapid analysis technique for use in the molecular diagnostics area and in life science. The overall performance of the inventive device, e.g. protein micro-array may be improved by tailoring the properties of the porous membrane support. Methods of chemically altering the total specific surface of said porous membranes in said devices resulting in surface functionalization allowing improved coupling of capture antibodies.

The examples illustrate the present invention in more detail, the present invention is, however not limited to these examples:

<u>Examples</u>

<u>Manufacture of the membranes according to the present invention</u>

Tetraethylene glycol dimethacrylate (TEGDMA) was purchased from Fluka Chemie (Steinheim, Germany), tripropylene glycol (TPG) from Sigma Aldrich Chemie GmbH (97% mixture of isomers, Steinheim, Germany) and 2,2 dimethoxy-1,2-phenylacetophenone (Irgacure 651) from Ciba Specialty Chemicals (Dilbeek, Basel, Switzerland). The monomer mixtures were prepared with 2 wt % of the photo initiator Irgacure 651, x wt % (with x between 15 wt % and 90 wt %) of the porogenic solvent tripropylene glycol, and (98-x) wt % of the cross-linking monomer TEGDMA. Diethylene glycol (DEG), triethylene glycol (TEG), tetraethylene glycol (TAEG) and polypropylene glycol (PPG) have been used as alternative porogenic solvents to perform control experiments. Some membranes were chemically functionalised by copolymerisation of glycidyl methacrylate (GMA, 2,3-epoxypropyl methacrylate, 97%, Steinheim, Germany) purchased from Sigma Aldrich Chemie GmbH in a 1:10 wt% ratio with respect to TEGDMA. Some membranes were grafted after polymerisation with diethylene triamine (DETA, Sigma Aldrich Chemie GmbH, Steinheim, Germany) at 60°C for 3 hours and subsequently kept in water for about 12 hours to remove the non-reacted TPG. All reagents were used as received.

The glass substrates of 5 x 5 cm$^2$ have a $SiO_2$ coating and were first cleaned gently with soap (Extran, neutral, Merck KGaA, Darmstadt, Germany) and water, subsequently rinsed with deionised water and blow-dried. Then, the glass substrates were cleaned in a UV ozone apparatus (PR 100, Ultra Violet Products) for 10 min. A homogeneous thin film of the polymerisation mixture with a thickness of about 50 $\mu$m was coated on a glass substrate by doctor blade coating process. The samples were exposed to UV light for 20 minutes if no further notification is given, using Philips PL 10 (2.6 mW/cm$^2$, $\lambda$= 320 - 420 nm, $\lambda_{max}$=365 nm). Prior to the UV exposure, the samples were flushed with $N_2$ for 3 minutes and the photo-polymerisation was also conducted in a protective $N_2$ atmosphere reducing polymerisation inhibition by $O_2$. After the polymerisation process was completed, the porogenic solvent and eventually remaining species were removed from the pores by washing in ethanol for 5 minutes. The membrane was finally blow-dried.

<u>Quantification of the total specific surface area of the inventive PIPS structure</u>

We have quantified the total specific surface area of our porous PIPS structures by BET adsorption measurements. Fig. 3 shows the evolution of the measured BET surface area with increasing TPG solvent concentration in the initial monomer mixture for three different UV light intensities during polymerisation. In the range between 50 and 60% porogenic solvent maximum surface areas (up to about 50 m$^2$/g) have been obtained, which can be considered as very large values for polymeric porous materials, which typically are between 8 and about 15 m$^2$/g.

<u>Determination of the binding recovery and efficiency of antibodies ink jet printed onto the PIPS membranes</u>

To find out how the inventive PIPS membranes are suited for immunoassays in comparison to a nitrocellulose membrane, the recovery and the efficiency of the binding of antibodies to different membranes have been determined. In these experiments nitrocellulose is taken into account as reference.

To determine the binding recovery of antibodies to the membranes, fluorophore labelled antibodies are printed on the membrane using an ink-jet printer. After printing, images are taken of the microarray using a LED-setup. The membranes are blocked with 1xPBS + 5% BSA for 1 hour at room temperature in the dark and are blow dried with $N_2$-gas for 30 seconds. The membranes are washed 3 x 5 minutes with 1 x PBS + Tween-20 at room temperature and are dried after washing, with the same procedure as used in the blocking step and images are taken. The images of the arrays before and after block and wash are processed in an Array-Pro Analyzer (MediaCybernetics, version 45). With these data the corrected intensity can be calculated using the following formula 1:

$$\text{Corrected Intensity} = \frac{(\text{signal intensity - background intensity})}{\text{Flash length } (\mu s) \cdot \text{amperage } (mA)} \qquad (\text{formula 1})$$

The flash length is the duration of the light pulse of the excitation light during which a picture is recorded. The amperage is the current at which the LED light is driven during this light pulse. The signal intensity is the measured fluorescent light intensity integrated over the spot. The background intensity is the acquired signal in a region next to the spot that is comparable in size.

The binding capacity of antibodies onto the different membranes is displayed in Fig. 4. Even after washing the membranes for two days, the recovery of antibodies from the membrane with 50% solvent, TEGDMA, GMA, and triamines stayed high. It is shown that the PIPS membranes with 50 v/v % with GMA and DETA yielded relatively high recovery (92%) as compared to the nitrocellulose membranes (40-80%). Also, the former membranes (50 v/v%, GMA, DETA) showed low membrane-to membrane variation (7%) as compared to nitrocellulose (variations of 39%). The efficiency experiments have only being performed once. The efficiency of antibodies printed on 50 v/v% solvent with GMA and DETA yielded an efficiency of 140%. In contrast, the membrane with 50 v/v% yielded two times lower efficiencies. Nitrocellulose yielded efficiencies in the range of 90-100%. The efficiencies over 100% may be explained by the fact that recovery was calculated using a different species as that for efficiency. We have seen that on the PIPIS membranes the recovery of different antibody species differ a lot as compared to nitrocellulose membranes.

To determine the binding efficiency of antibodies onto the membranes, capture antibodies are printed on the membranes using an ink-jet printer. Also fluorescently labeled antibodies in various concentrations are printed, which act as an internal calibrator. We use the internal calibrator to normalize signal intensity such that assay performance on different membranes can be evaluated. After printing the membranes are blocked with 1xPBS + 5% BSA for 1 hour at room temperature in the dark. The blocking buffer is removed and the membranes are incubated with fluorophore labelled detection antibodies for 1 hour in the dark at room temperature. After incubation the membranes are washed 3 x 5 minutes with 1 x PBS + Tween-20 at room temperature and are dried thereafter with nitrogen gas, after which images are taken. The images of the arrays are processed in an Array-Pro Analyzer (MediaCybernetics, version 45). With these data the binding efficiency can be calculated as follows.

From the gathered data the recovery is calculated by formula 2.

$$\text{Recovery} = \frac{\text{Corrected Intensity}_{\text{after blocking \& washing}}}{\text{Corrected Intensity}_{\text{before blocking \& washing}}} \cdot 100\% \qquad \text{(Formula 2)}$$

Then the amount of molecules per $\mu m^2$ is calculated according to the internal calibration curve and the analyte using formula 3.

$$\text{Molecules}\Big/_{\mu m^2} = (\frac{n_{dr} \cdot V_{dr} \cdot c \cdot N_a}{0.25 \cdot \pi \cdot (d^2)} / 100) * \text{Recovery} \qquad \text{(Formula 3)}$$

$n_{dr}$ = Number of droplets printed
$V_{dr}$ = Volume of the droplets (L)
c. = concentration printed (mol/L)
$N_a$ = Avogadro Number $6.0223 \times 10^{23}$
d = diameter ($\mu$m)

The corrected intensity is determined from the data of an Array-Pro Analyzer (MediaCybernetics, version 45) and an in-house computer program for calculating the recovery as well as the corrected intensity. The corrected intensity coming from the internal calibrators is set against the number of molecules/$\mu m^2$ bound to the membrane. This calibration curve is used to calculate the number of molecules/$\mu m^2$ present on the capture spots. The corrected intensity of the analyte is entered in the formula of the graph, with that the amounts of molecules are calculated. To calculate the binding efficiency formula 4 is used. Note that the number of molecules/$\mu m^2$ is multiplied by 2, because there are two antigen binding sites per antibody.

$$\text{Effeciency} = \frac{\text{Molecules}\Big/_{\mu m^2} \text{Ag bound to the array}}{\text{Molecules}\Big/_{\mu m^2} \text{Capture Ab present on the array} * 2} \cdot 100\% \qquad \text{(Formula 4)}$$

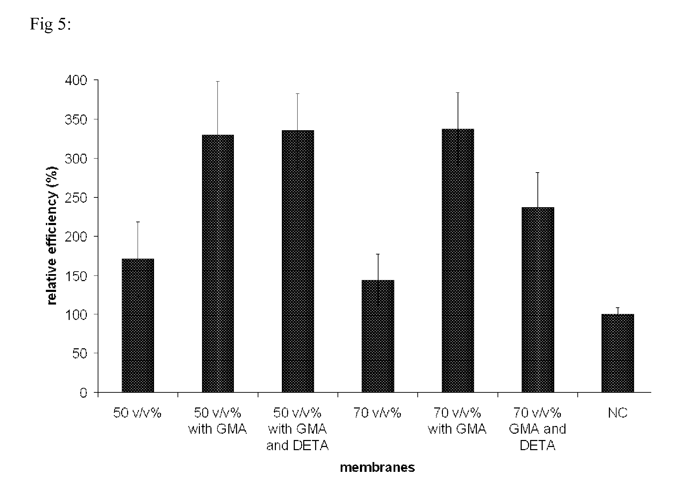

The binding efficiency of antibodies onto the different membranes is shown in Fig. 5.

The efficiency in Fig. 5 shows that the PIPS membranes yield higher efficiencies than nitrocellulose membranes.

## Claims

1. A porous essentially isotropic polymeric membrane for use in a flow-through/flow-over immunoassay, wherein the average total specific surface area is greater than 20 m$^2$/g and wherein the average pore size distribution (FWHM) is $\leq$ twice the average pore size.

2. A porous essentially isotropic polymeric membrane according to claim 1, wherein the total specific surface area is between 30 and 55 m$^2$/g.

3. A porous essentially isotropic polymeric membrane according to claim 1 or 2, wherein the pore size is between 10 nm and 10 $\mu$m.

4. A porous essentially isotropic polymeric membrane according to claims 1 to 3, wherein the membrane thickness is in the range between 10 and 300 $\mu$m.

5. A porous essentially isotropic polymeric membrane according to claims 1 to 4 obtainable by photo-polymerization induced phase separation of a mixture comprising a cross-linking monomer and a single porogenic solvent, wherein the solvent concentration is between 40 and 80 wt%.

6. A porous essentially isotropic polymeric membrane according to claim 5, wherein the cross-linking monomer is tetraethyleneglycoldimethylacrylate.

7. A porous essentially isotropic polymeric membrane according to any of claims 5 or 6, wherein the solvent is either polypropyleneglycol or tripropyleneglycol.

8. A porous essentially isotropic polymeric membrane according to claims 1 to 7, wherein the total specific surface of said porous membrane is chemically altered resulting in surface functionalization.

9. A porous essentially isotropic polymeric membrane according to claims 1 to 8, wherein capture antibodies are coupled to the functionalized surface.

10. A method of making a membrane according to claims 1 to 9 comprising the steps of:

    - providing a mixture comprising a cross-linking monomer and a single porogenic solvent, wherein the solvent concentration is between 40 and 80 wt%,
    - performing a photo-polymerization induced phase separation process applying UV light,
    - after completion of polymerization, rinsing the porous membrane with a second solvent, and
    - drying of the porous membrane.

11. A method of making a membrane according to claim 10, wherein the cross-linking monomer is tetraethyleneglycoldimethylacrylate.

12. A method of making a membrane according to claims 10 or 11, wherein the single porogenic solvent is either polypropyleneglycol or tripropyleneglycol.

13. A method of making a membrane according to claims 10 to 12, wherein in a subsequent post-polymerization step the total specific surface of said porous membrane is chemically altered resulting in surface functionalization.

14. A method of making a membrane according to claims 10 to 13, wherein capture antibodies are coupled to the surface of the membrane.

15. A device comprising a porous essentially isotropic polymeric membrane according to claims 1 to 9.

16. A micro fluidic bio-separation device comprising a plurality of porous elements made of a porous essentially isotropic polymeric membrane according to any of claims 1 to 9 integrated in at least a part of a channel or in multiple parts of multiple channels of said micro fluidic device.

17. The use of a membrane according to any of claims 1 to 9 and the use of a device according to claims 15 or 16 for molecular diagnostics.

18. The use of a membrane according to any of claims 1 to 9 and the use of a device according to claims 15 or 16 for detections of proteins in bodily fluids.

19. The use of a membrane according to any of claims 1 to 9 and the use of a device according to claims 15 or 16 for performing a quantitative immunoassay for measuring protein concentration in an analyte solution.

Fig 1:

Fig 2:

Fig 3:

Fig 4:

Fig 5:

**European Patent Office**

**EUROPEAN SEARCH REPORT**

Application Number

EP 07 12 0468

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | WO 2007/069114 A (KONINKL PHILIPS ELECTRONICS NV [NL]; KURT RALPH [NL]; BROER DIRK JAN []) 21 June 2007 (2007-06-21) * page 4, line 11 - page 5, line 11; claims 1-10 * | 1-19 | INV. C08J5/22 G01N33/50 |
| X | US 2006/228386 A1 (STEPHENS CHRIS [US] ET AL) 12 October 2006 (2006-10-12) * claims 1-20; examples 1,2 * | 1-19 | |
| D,X | SEBASTIAAN EELTING ET AL.: "Tailoring the Morphology of Methacrylate Ester-Based Monoliths for Optimum Efficiency in Liquid Chromatography" ANAL. CHEM., vol. 77, no. 22, 15 November 2005 (2005-11-15), pages 7342-7347, XP002477049 * page 7344 * | 1-19 | |
| D,X | COURTOIS ET AL: "Novel monolithic materials using poly(ethylene glycol) as porogen for protein separation" POLYMER, ELSEVIER SCIENCE PUBLISHERS B.V, GB, vol. 47, no. 8, 5 April 2006 (2006-04-05), pages 2603-2611, XP005365912 ISSN: 0032-3861 * table 2 * | 1-19 | TECHNICAL FIELDS SEARCHED (IPC) C08J G01N C08F |

-/--

The present search report has been drawn up for all claims

| Place of search | Date of completion of the search | Examiner |
|---|---|---|
| Munich | 18 April 2008 | olde Scheper, Bernd |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number

EP 07 12 0468

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BETINA MAYR ET AL.: "Methathesis-Based Monoliths: Influenece of Polymerisation Conditions on the Separation of Biomolecules" ANAL.CHEM., vol. 73, no. 17, 1 September 2001 (2001-09-01), pages 4071-4078, XP002477050 * table 1 * | 1-19 | |
| A | SEBASTIAAN EELTINK ET AL.: "Controlling thesurface chemistry and chromatographic properties of methacrylate-ester-based monolithic capillary columns via photografting" J.SEP.SCI., vol. 30, no. 3, 13 February 2007 (2007-02-13), pages 407-413, XP002477051 * the whole document * | 1-19 | |
| D,A | CAMILLA VIKLUND ET AL.: "Monolithic, "Molded", Porous Materials with High Flow Characteristics for Separations, Catalysis, or Solid-Phase Chemistry: Control of Porous Properties during Polymerisation" CHEM. MATER., vol. 8, March 1996 (1996-03), pages 744-750, XP002477052 * the whole document * | 1-19 | TECHNICAL FIELDS SEARCHED (IPC) |

The present search report has been drawn up for all claims

| Place of search | Date of completion of the search | Examiner |
|---|---|---|
| Munich | 18 April 2008 | olde Scheper, Bernd |

EPO FORM 1503 03.82 (P04C01)

## ANNEX TO THE EUROPEAN SEARCH REPORT
## ON EUROPEAN PATENT APPLICATION NO.

EP 07 12 0468

This annex lists the patent family members relating to the patent documents cited in the above-mentioned European search report.
The members are as contained in the European Patent Office EDP file on
The European Patent Office is in no way liable for these particulars which are merely given for the purpose of information.

18-04-2008

| Patent document cited in search report | Publication date | Patent family member(s) | Publication date |
|---|---|---|---|
| WO 2007069114 A | 21-06-2007 | NONE | |
| US 2006228386 A1 | 12-10-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5450516 A
- US 5725949 A
- US 6214232 B
- US 6225131 B
- US 5741647 A
- US 6020187 A
- WO 03004162 A
- WO 2005077537 A1
- US 20050106583 A1
- US 6893879 B
- WO 2005069004 A
- WO 2007069114 A
- US 4224415 A
- WO 05077537 A1
- US 6893879 B2
- WO 9511755 A
- US 20050069937 A
- US 20060003381 A
- WO 9322678 A

### Non-patent literature cited in the description

- **HJERTEN, S. ; LIAO, J. L. ; ZHANG, R.** *J. Chromatogr.,* 1989, vol. 473, 273-275
- **TENNIKOVA, T. B. ; SVEC, F. ; BELENKII, B. G.** *J. Liquid Chromatogr.,* 1990, vol. 13, 63-70
- **SVEC, F. ; FRECHET, J. M. J.** *Anal. Chem.,* 1992, vol. 54, 820-822
- **GUSEV, I. ; HUANG, X. ; HORVATH, C.** *J. Chromatogr. A,* 1999, vol. 69, 4499-4507
- **MINAKUCHI, H. ; NAKANISHI, K. ; SOGA, N. ; ISHIZUKA, N. ; TANAKA, N.** *Anal. Chem.,* 1996, vol. 68, 3498-3501
- **S. EELTINK ; E.F. HILDER ; L. GEISER ; F. SVEC ; J.M.J. FRECHET ; G.P. ROZING ; P.J. SCHOENMAKERS ; W.T. KOK.** *Journal of Separation Science,* 2007, 30
- Monolithic Materials: Preparation, Properties, and Applications. Elsevier, 2003
- **SVEC, F.** *J. Sep. Sci.,* 2005, vol. 28, 729-745
- **SVEC, F. ; HUBER, C. G.** *Anal. Chem.,* 2006, vol. 78, 2100-2107
- **ALLEN, D. ; EL RAZZI, Z.** *Electrophoresis,* 2003, vol. 24, 3962-3976
- **RIEUX, L. ; NIEDERLANDER, H. ; VERPOORTE, E. ; BISCHOFF, R.** *J. Sep. Sci.,* 2005, vol. 28, 1628-1641
- **VIKLUND, C. ; SVEC, F. ; FRECHET, J. M. J. ; IRGUM, K.** *Chem. Mater.,* 1996, vol. 8, 744-750
- **PETERS, E. C. ; PETRO, M. ; SVEC, F. ; FRECHET, J. M. J.** *Anal. Chem.,* 1998, vol. 70, 2288-2295
- **EELTINK, S. ; HERRERO-MARTINEZ, J. M. ; ROZING, G. P. ; SCHOENMAKERS, P. J. ; KOK, W. TH.** *Anal. Chem.,* 2005, vol. 77, 7342-7347
- **COURTOIS, J. ; BYSTOM, E. ; IRGUM, K.** *Polymer,* 2006, vol. 47, 2603-2611
- **ROHR, T. ; HILDER, E. F. ; DONOVAN, J. J. ; SVEC, F. ; FRECHET, J. M. J.** *Macromolecules,* 2003, vol. 36, 1677-1684
- **ROHR, T. ; OGLETREE, F. ; SVEC, F. ; FRECHET, J. M. J.** *Adv. Funct. Mater.,* 2003, vol. 13, 264-270
- **MEYER, U. ; SVEC, F. ; FRECHET, J. M. J. ; HAWKER, C. J. ; IRGUM., K.** *Macromolecules,* 2000, vol. 33, 7769-7775
- **PETERSON, D. S. ; ROHR, T. ; SVEC, F. ; FRECHET, J. M. J.** *Anal. Chem.,* 2003, vol. 75, 5328-5635
- **PUCCI, V. ; RAGGI, M. A. ; SVEC, F. ; FRECHET, J. M. J.** *J. Sep. Sci.,* 2004, vol. 27, 779-788
- **PENZOL et al.** *Biotechnol. Bioeng.,* 1998, vol. 60, 518-523